# EUROPEAN PATENT APPLICATION

(11) **EP 3 901 270 A1**
(43) Date of publication of application: **27.10.2021**
(21) Application number: 20305376.4
(22) Date of filing: 20.04.2020
(51) Int. Cl.: C12P 7/40, C12P 7/52, C12N 1/20, C12R 1/02

(54) **METHOD FOR PRODUCING ORGANIC ACIDS BY BIOCONVERSION**

(71) Applicant: Institut national de recherche pour l'agriculture, l'alimentation et l'environnement, 75007 Paris (FR); Institut des sciences et industries du vivant et de l'environnement, 75005 Paris (FR)
(72) Inventor: de Salivet de Fouchécour, Florence, 78850 Thiverval-Grignon (FR); Sanchez Castaneda, Ana Karen, 92190 Clamart (FR); Spinnler, Henry Eric, 92310 Sèvres (FR); Athès-Dutour, Violaine, 78330 Fontenay le Fleury (FR); Moussa, Marwen, 78130 Les Mureaux (FR); Bérion, Claire, 78370 Plaisir (FR); Trelea, Ioan Cristian, 78530 BUC (FR)
(74) Representative: Ipside

(57) **Abstract**

The invention relates to a method for the production of a carboxylic acid from a primary alcohol, said method comprising: a first step of growing a strain of bacterium of the genus *Acetobacter* capable of selectively oxidizing said alcohol into said carboxylic acid, in an appropriate growth medium comprising glycerol as carbon source, until said bacterium reaches a late exponential growth phase; then a selective oxidation step comprising aerobically culturing said bacterium in a bioconversion reactor containing an appropriate liquid bioconversion medium containing said alcohol and glycerol, for a sufficient time to oxidize said alcohol into said carboxylic acid.

## Description

The invention lies in the field of the production of organic compounds by biological processes.

More particularly, the invention relates to a method for the production of a carboxylic acid from a primary alcohol, by bioconversion carried out by a microorganism of the genus *Acetobacter.*

Because petrochemical resources are bound to become scarce and their use was proven to contribute to global warming, it has become a necessity to reduce dependency on non-renewable feedstocks and to start a transition towards a more sustainable, biobased economy. Therefore, biotechnological processes for bulk chemical production have been gaining great interest over the past decades, as a solution to replace existing chemical processes based on fossil feedstocks.

In order to promote research in this field, the US Department of Energy published a list of the most promising building-blocks that can be obtained from biomass. Thanks to its two functional groups (carboxyl and β-hydroxyl), 3-hydroxypropionic acid (3-HP, CAS 503-66-2) is a versatile molecule and has great potential for further transformation into useful chemicals. 3-hydroxypropionic acid was therefore included by the US Department of Energy in the top value-added chemicals list. 3-hydroxypropionic acid applications can be divided into three categories: direct use, polymerization, and conversion to other value-added chemicals. 3-hydroxypropionic acid can be used directly by the food and feed industry as an additive or as a preservative agent. It can also be polymerized to poly(3-hydroxypropionate) or to 3-hydroxypropionic acid containing co-polymers. In particular, poly(3-hydroxypropionate) has very promising properties and may become one alternative to fossil fuel-derived plastics. Lastly, 3-hydroxypropionic acid can also be converted to numerous other useful chemicals, among which acrylic acid is the most noteworthy.

Many other organic acids have a great economic interest.

Apart from chemical synthesis, organic acids may be prepared in several ways: they may be isolated from natural sources, or obtained by microbiological fermentation.

In particular, a lot of research work has been carried out for the production of 3-hydroxypropionic acid by bioconversion processes. Many process engineering strategies have been developed in this field. Some of them are for example discussed in the publication from de Fouchecour et al., 2018, Biotechnology Advances 36: 1207-1222.

One of the dominant approaches proposed so far is the biosynthesis of 3-hydroxypropionic acid from glycerol, either through a coenzyme A-dependent or coenzyme A-independent metabolic route. In both cases, glycerol is first converted into 3-hydroxypropionaldehyde, which is subsequently oxidized into 3-hydroxypropionic acid or reduced into 1,3-propanediol. Among the microorganisms that can naturally produce 3-hydroxypropionic acid from glycerol, *Lactobacillus reuteri* is able to perform the bioconversion of glycerol into the sole 3-hydroxypropionic acid and 1,3-propanediol, with no other side products. These oxidative and reducing pathways are in redox balance with one another. 1,3-propanediol is therefore an obligate co-product of 3-hydroxypropionic acid biosynthesis from glycerol. Consequently, it is necessary to implement a subsequent step for its conversion to 3-hydroxypropionic acid, in order to improve the yield and the selectivity of the overall process of production of 3-hydroxypropionic acid.

Acetic acid bacteria are capable of oxidizing primary alcohols into carboxylic acids. It has been proposed by the prior art, illustrated for example by the publication of Li et al., 2016, Biotechnology and Bioprocess Engineering 21: 523-530, to take advantage of the high efficiency of alcohols oxidation property of the bacterium *Acetobacter* sp. CGMCC 8142 for biosynthesizing 3-hydroxypropionic acid from 1,3-propanediol. However, the productivity of the reaction is low, of about 0.9 g/(L.h).

The present invention aims to overcome the drawbacks of the methods for the bioconversion of 1,3-propanediol into 3-hydroxypropionic acid proposed by the prior art, in particular as described above, by proposing a method for biologically producing 3-hydroxypropionic acid from 1,3-propanediol and, more generally, a carboxylic acid from the corresponding alcohol, such a method implementing a non-pathogenic, "Generally Recognized As Safe" (G.R.A.S.), microorganism, for realizing the bioconversion of the alcohol into the acid with a high productivity and a high reaction yield.

Other objectives of the invention are that this method may be implemented at low cost, and that it makes it possible to obtain the acid with a high degree of purity.

The present inventors have now developed a method for the production of a carboxylic acid from a primary alcohol that meets these objectives.

The method of the invention comprises:
- a first step, that will be qualified as a "growth step" in the present description, of growing a strain of a bacterium of the genus *Acetobacter* capable of selectively oxidizing said alcohol into said carboxylic acid, in an appropriate growth medium comprising glycerol as carbon source, until said bacterium reaches a late exponential growth phase;
- then a selective oxidation step, also called bioconversion step, comprising aerobically culturing said bacterium, having reached the late exponential growth phase, in a bioconversion reactor containing an appropriate liquid bioconversion medium containing said alcohol, for a sufficient time to oxidize said alcohol into said carboxylic acid, via the corresponding intermediary aldehyde. Said bioconversion medium contains glycerol.

A primary alcohol is herein defined, in a conventional way, as an alcohol wherein at least one hydroxyl group is connected to a primary carbon atom.

It has been discovered by the inventors that, entirely surprisingly, the sequential strategy of the invention makes it possible to obtain the carboxylic acid with a very high yield, more precisely with a molar yield close to 100 %, and a particularly high productivity, of several grams per liter of bioconversion medium per hour, and even of more than 4 g.L⁻¹.h⁻¹. An acid titer as high as 60 g/L can furthermore be obtained in the fermentation medium.

The performance of the method of the invention is much higher than that of the other methods of the prior art which implement a non-pathogenic microorganism, like the method of the invention.

First, the sequential method of the invention, comprising a first step of growing the bacterium, and a subsequent step of performing the bioconversion / selective oxidation reaction, by supplying the alcohol to the reactor containing the grown bacterium, achieves a more efficient acid biosynthesis, in particular a much higher specific productivity and a much higher acid titer, than growth-coupled bioconversion processes, wherein growth of the bacterium and bioconversion are carried out simultaneously.

Furthermore, the person skilled in the art would have thought that the presence of glycerol in the bioconversion medium would have been detrimental to the bioconversion of the alcohol into the carboxylic acid, and would therefore have ensured that the bioconversion medium did not contain glycerol. This is indeed what has been done in the work described in the aforementioned publication of Li et al. On the contrary, it has been discovered by the inventors that it is quite the opposite, and that the presence of glycerol in the bioconversion medium not only is not detrimental to this bioconversion reaction, but that it even increases the bioconversion performance. This result was completely unexpected from the prior art and the general knowledge of the person skilled in the art.

By "appropriate growth medium", and by "appropriate bioconversion medium", it is meant in the present description sterile aqueous media containing the nutrients that are essential or beneficial to the growth, respectively to the maintenance, of the bacterium. Such media typically contain a carbon source, which according to the invention is at least partly formed of glycerol, a nitrogen source, for example peptone, yeast extract, urea, an ammonium salt and/or ammonia NH₃, and a phosphorus source, for example monopotassium phosphate or dipotassium phosphate. They may additionally contain trace elements, such as metal salts, and/or other factors, such as amino acids, vitamins, etc. It is within the skills of the person skilled in the art to be able to prepare such appropriate growth medium and bioconversion medium for each given strain of bacterium of the genus *Acetobacter* he wishes to implement.

According to the method of the invention biomass is first produced on glycerol in batch mode, during a sufficient time to obtain late exponential growth phase. It falls within the skills of the person skilled in the art to know how to determine the moment when the bacterium has reached the late exponential growth phase. By definition, the late exponential phase corresponds to the period when the natural logarithm of the biomass concentration is not a linear function of time anymore. The moment when the bacterium has reached the late exponential growth phase can for example be determined by measuring the optical density at 600 nm of the growth medium containing the bacterium.

The duration of the growth step may be comprised between 24 and 48 h.

At the end of this primary growth step, it is observed that no organic acid, that could otherwise have competed with the alcohol in the subsequent selective oxidation step, has been produced and has accumulated in the growth medium.

Bacteria of the genus *Acetobacter* are acetic acid bacteria, Gram-negative, obligate aerobes, comprised in the *Acetobacteraceae* family. They possess various membrane-bound dehydrogenases, which gives them the ability to oxidize a very broad range of alcohols directly in the periplasm, to form the corresponding aldehydes, which are then further oxidized into the corresponding carboxylic acids by an aldehyde dehydrogenase. It is within the skills of the person skilled in the art, according to the particular alcohol he seeks to oxidize, to select the appropriate strain of the genus *Acetobacter* capable of selectively performing this oxidation.

By "capable of selectively oxidizing said alcohol into said carboxylic acid", it is herein meant that the bacterium oxidizes the alcohol selectively into the carboxylic acid, i.e. that the carboxylic acid is the only final product of the oxidation reaction.

In particular, when the alcohol is a diol, comprising two hydroxyl groups carried by primary carbon atoms, a single carboxylic acid is obtained by the method of the invention, generally a hydroxy-acid.

For the purpose of identifying bacteria of the genus *Acetobacter* capable of selectively oxidizing a primary alcohol of interest into a targeted carboxylic acid, the person skilled in the art may refer to the works published by the prior art in this field, and/or carry out bioconversion tests on small quantities of the alcohol of interest in order to determine which oxidation product(s) is/are obtained.

By way of example, the strain available in the Biological Resource Center of the Pasteur Institute under accession number CIP 58.66, may advantageously be used for the selective bioconversion into a carboxylic acid of many alcohols, in particular of 1,3-propanediol into 3-hydroxypropionic acid.

In the selective oxidation step of the method of the invention, the bacterial cells in late exponential growth phase, supplied with the alcohol, and in the presence of glycerol, quickly oxidize the alcohol into the corresponding aldehyde, then oxidize the latter into the corresponding carboxylic acid. The duration of the selective oxidation step, which is sufficient to oxidize said alcohol into said carboxylic acid, is determinable by the person skilled in the art, in particular according to the amount of alcohol provided in the bioconversion medium. The person skilled in the art may for that purpose collect samples of the bioconversion medium at different times of the bioconversion reaction, and determine the amounts of residual alcohol and of produced acid contained therein, for example by chromatography analysis, in particular to gas chromatography coupled to mass spectrometry, and comparison of the results with predetermined calibration curves.

In particular embodiments of the invention the selective oxidation step is carried out for at least 4 hours. Such a duration generally ensures that at least part of the alcohol present in the bioconversion medium has been converted into the corresponding acid. When the bioconversion medium contains high amounts of the alcohol, a duration of the selective oxidation step of 48 hours generally allows for the totality of the alcohol to be converted into the acid.

The duration of the selective oxidation step may thus be comprised between 4 and 48 hours, in particular between 4 and 25 hours.

During the selective oxidation step, no final accumulation of intermediary aldehyde is observed in the bioconversion reactor. This is all the more advantageous that such intermediary aldehyde accumulation would be detrimental to the bacterial cells. A secondary exponential growth phase is observed in the bioconversion medium, without any latency phase. It is presumed that the glycerol and the alcohol present in the bioconversion medium both serve as a carbon source for the strain's growth. The alcohol is advantageously quantitatively converted into the corresponding acid.

The method according to the invention may further respond to one or more of the features described below, implemented individually or in each of their technically operating combinations.

The strain implemented in the method of the invention may belong to any of the species *Acetobacter aceti*, *Acetobacter peroxydans*, *Acetobacter tropicalis*, *Acetobacter syzygii*, *Acetobacter okinawensis*, *Acetobacter lambici*, etc. It may otherwise be designated as *Acetobacter* sp.

In particular embodiments of the invention, the strain implemented in the method of the invention is the strain *Acetobacter* sp. available at the Biological Resource Center of the Pasteur Institute (Paris) under accession number CIP 58.66. Although this strain is presented as belonging to the species *Acetobacter aceti*, it has been established by the inventors, by 16S rRNA analysis, that it does not really belong to that species, and should be qualified as *Acetobacter* sp.

The strain of the species *Acetobacter aceti* available at the Biological Resource Center of the Pasteur Institute (Paris) under accession number CIP 103.11 may also be used in the method of the invention, in particular for the bioconversion of 1,3-propanediol into 3-hydroxypropionic acid.

The bacterium may be a wild-type bacterium or a genetically-modified, recombinant bacterium.

Preferably, the bacterium of the genus *Acetobacter* is the sole microorganism present in the growth medium, then in the bioconversion medium. A single strain of the genus *Acetobacter,* or a mixture of such strains, all being capable of selectively oxidizing the alcohol into the carboxylic acid, may be used.

In particular embodiments of the invention, the growth medium contains an initial concentration of between 10 and 20 g/L of glycerol.

The pH of the growth medium is preferably between 3.9 and 6.9, for example of about 5. The pH of the growth medium can for example be adjusted with sulfuric acid.

The growth step is preferably performed:
- under stirring, with a stirring rate preferably comprised between 100 and 800 rotations per minute, using a radial flow impeller, such as a Rushton turbine, placed inside the reactor, or of about 200 rotations per minute, the reactor being placed inside a shaking incubator;
- and/or with aeration of the bioconversion medium at an aeration rate comprised between 1 and 4 NL/min.

The growth step is preferably carried out: at 30 °C; and/or under a partial dioxygen pressure pO₂ of at least 5 %, for example of between 10 and 40 %.

In preferred embodiments of the invention the bioconversion medium contains a concentration of between 5.5 and 20 g/l of glycerol.

The method of the invention may comprise a step of verifying the presence of glycerol in the fermentation medium; and optionally, when necessary, a step of adding glycerol in the fermentation medium in order to restore a suitable glycerol concentration therein.

The selective oxidation step is preferably performed:
- under stirring, with a stirring rate preferably comprised between 100 and 800 rotations per minute, using a radial flow impeller, such as a Rushton turbine, placed inside the reactor, or of about 200 rotations per minute, the reactor being placed inside a shaking incubator;
- and/or with aeration of the bioconversion medium at an aeration rate comprised between 1 and 4 NL/min.

The selective oxidation step is preferably carried out: at 30 °C; and/or under a partial dioxygen pressure pO₂ of at least 4 %.

In preferred embodiments of the invention, the bioconversion medium contains between 0.5 and 10 g/L, preferably between 2 and 5 g/L, of the alcohol to be biologically oxidized into the carboxylic acid. Such an amount of the alcohol in the bioconversion medium is associated to the highest performance of the method of the invention.

The selective oxidation step of the method of the invention may comprise batch-feeding the alcohol into the bioconversion reactor. The feeding of the alcohol into the bioconversion reactor may otherwise be carried out continuously.

In all cases, the feeding rate is preferably adjusted to the metabolic activity of the bacterium.

The method of the invention may for example comprise a plurality of steps of batch-feeding the alcohol into the bioconversion reactor, an amount of 0.5 to 10 grams, preferably 2 to 10 grams, of alcohol per liter of bioconversion medium being supplied to the bioconversion medium at each feeding step. These successive batch-feeding steps may be performed at 3.5 to 5 hours intervals.

In particular embodiments of the invention, the method comprises adjusting the pH of the fermentation medium at a value comprised between 4 and 5. Sulfuric acid may for example be used for this purpose, at the beginning of the selective oxidation step. A basic compound may subsequently be used to maintain the pH of the bioconversion medium into the desired range as the oxidation progresses, in order to counterbalance with the increase in the concentration of carboxylic acid in the medium.

The alcohol that the method of the invention aims at converting into a carboxylic acid may be a mono-alcohol, a diol or a polyalcohol. It may be selected in the group consisting of:
- 1,3-propanediol, for producing 3-hydroxypropionic acid,
- 2-methylbutanol, for producing 2-methylbutyric acid,
- 2-phenylethanol, for producing phenylacetic acid,
- butanol, for producing butyric acid,
- 3-methylbutanol, for producing 3-methylbutyric acid,
- 1,4-butanediol, for producing 4-hydroxybutyric acid,
- 1,4-pentanediol, for producing 4-hydroxyvaleric acid,
- and hexanol, for producing hexanoic acid.

All of these alcohols are successfully converted into the corresponding carboxylic acids / hydroxy-acids by the above-mentioned *Acetobacter* sp. strain CIP 58.66.

When the alcohol to be converted is a diol, the hydroxyl groups of which are carried by carbon atoms which are separated from one another by 2 atoms, and when the carboxylic acid formed by the bioconversion reaction is a hydroxy-acid, there can advantageously occur in the bioconversion medium, according to the operating conditions, a cyclization of hydroxy-acid molecules into a lactone. Such a cyclization step can for example be observed for 4-hydroxybutyric acid produced from 1,4-butanediol, resulting in the obtention of γ-butyrolactone; or for 4-hydroxyvaleric acid produced from 1,4-pentanediol, resulting in the obtention of γ-valerolactone. Such lactones are in particular useful in the field of aromas.

In preferred embodiments of the invention, the method comprises the extraction of said carboxylic acid from the bioconversion medium.

The extraction is preferably carried out continuously, as the carboxylic acid is formed in the bioconversion medium. Such an embodiment of the method of the invention advantageously increases the bioconversion performance, as it avoids accumulation in the bioconversion medium of the carboxylic acid, which is toxic to the bacterial cells. In such an embodiment, adjustment of the pH of the bioconversion medium is not necessary, as the pH is auto-regulated by the continuous removal of the carboxylic acid from the bioconversion medium.

The extraction is preferably carried out outside the bioconversion reactor.

In preferred embodiments of the invention, the extraction of the carboxylic acid from the bioconversion medium is a liquid-liquid reactive extraction, using an amine-containing organic extracting solution. Alternatively, a liquid-liquid extraction may be carried out with a phosphorus-bonded oxygen-bearing extractant such as tributyl phosphate or trioctylphosphine oxide.

A complex is formed at the interface between the organic extracting phase and the aqueous bioconversion phase, between the carboxylic acid and the amine. This complex being hydrophobic, the carboxylic acid is extracted from the aqueous phase into the organic phase. This acid-base interaction only occurs between the amine and the carboxylic acid, so that the extraction step does not interfere with the bioconversion reaction. The concentrations of the alcohol to be converted and of the glycerol in the bioconversion medium are in particular not affected by the extraction step.

The amine contained in the extraction solution is preferably an aliphatic amine.

The extracting solution may for example comprise a tertiary amine chosen among didodecylmethylamine (DDMA), trioctylamine (TOA), Alamine 336 (mixture of tertiary amines with chain lengths of 8-10 carbon atoms) or tridecylamine, or a mixture of such compounds.

The amine of the extracting solution is contained in an organic solvent or a mixture of organic solvents. These solvents are chosen to be non-miscible with the aqueous bioconversion medium and chemically inert with respect to the carboxylic acid, the alcohol and glycerol. The organic solvent(s) is/are for example chosen from dodecanol, octanol, decanol, oleyl alcohol, 2-butyl-1-octanol, 2-hexyl-1-decanol, dodecane, decane, or a mixture thereof.

An example of extracting solution that may be used in the method of the invention contains 20 % (v/v) of didodecylmethylamine diluted in 40 % (v/v) dodecanol and 40 % (v/v) dodecane.

The extracting solution is of course chosen so as to be the least toxic possible for the *Acetobacter* bacterium.

In preferred embodiments of the invention, the liquid-liquid reactive extraction is carried out through a membrane, preferably in a hollow-fibre membrane contactor.

The contact of the bioconversion medium and the extracting solution then occurs in the membrane, in the pores of the latter. The membrane is preferably hydrophobic, so that the bioconversion medium does not penetrate into its pores. Such an embodiment advantageously reduces the impact of the extraction step on the production of the carboxylic acid by the bacterium, and the bacterium metabolism

In preferred embodiments of the invention, the extraction is carried out continuously, outside the bioconversion reactor, in a membrane contactor. Such an embodiment achieves the best extraction performance. It has been discovered by the inventors that such an extraction method, known as pertraction, is advantageously compatible with the activity of the bacteria of the genus *Acetobacter,* in particular with its high oxidative capacities. The extraction step allows for the *in situ* / in stream extraction of the totality of the carboxylic acid produced, with an extraction flow of 1.1 g of acid.m⁻².h⁻¹, that is compatible with the speed of bacterial conversion by the bacteria of the genus *Acetobacter.* A rapid and efficient selective removal of the carboxylic acid from the diluted bioconversion medium is achieved, even at low acid concentrations.

Furthermore, such an embodiment of the method of the invention makes it possible to recover the carboxylic acid obtained from the bioconversion reactor at reduced cost.

In preferred embodiments of the invention, the membrane is of the hollow-fibre type. The membrane then offers a particularly high interfacial area, which advantageously improves both the transfer of the carboxylic acid from the bioconversion medium to the extracting solution and the compactness of the overall device used for implementing the method of the invention.

The exchange surface of the membrane is preferably of between 0.4 and 1.6 m², for example of between 0.4 and 1.2 m².

In particular embodiments the method comprises a further step of recovering the carboxylic acid from the extracting solution, preferably continuously, as the extracting solution withdraws the carboxylic acid from the bioconversion medium.

This step may be carried out by liquid-liquid reactive extraction, for example by means of an aqueous phase containing a stronger base than the amine of the extracting solution, such as sodium hydroxide. This further step, also referred to as a back-extraction step, realizes at the same time the recovery of the carboxylic acid, in the form of a salt, and the regeneration of the extracting solution. The regenerated extracting solution may thus be re-used in the extraction step of the method of the invention. A lower quantity of extracting solution is then necessary to carry out this extraction step.

The back-extraction step is preferably carried out through a membrane, preferably in a hollow-fibre membrane contactor.

This overall process of combined extraction and back-extraction makes it possible to recover the carboxylic acid produced in the bioconversion reactor at a high degree of purity, in the form of a salt in a concentrated aqueous solution.

This overall process can be carried out at low cost, using any suitable device, in particular a device equipped with two hollow-fibre membrane contactors. It proves of great industrial interest.

In particular embodiments of the invention, the method also comprises a preliminary step of bioproducing said alcohol by a micro-organism, for example from glycerol by *Lactobacillus reuteri* to produce 1,3-propanediol; and a step of feeding the alcohol thus produced into said bioconversion medium, without any intermediate purification step. It is meant here that said alcohol fed into the bioconversion reactor of the invention is obtained by simply collecting supernatant from the micro-organism culture medium, said supernatant being fed in the bioconversion reactor implemented according to the invention.

The method of the invention then advantageously forms an entirely biological process, which is simple and quick to implement.

For example, it makes it possible to obtain 3-hydroxypropionic acid from biobased glycerol at low cost and highly environmentally-friendly.

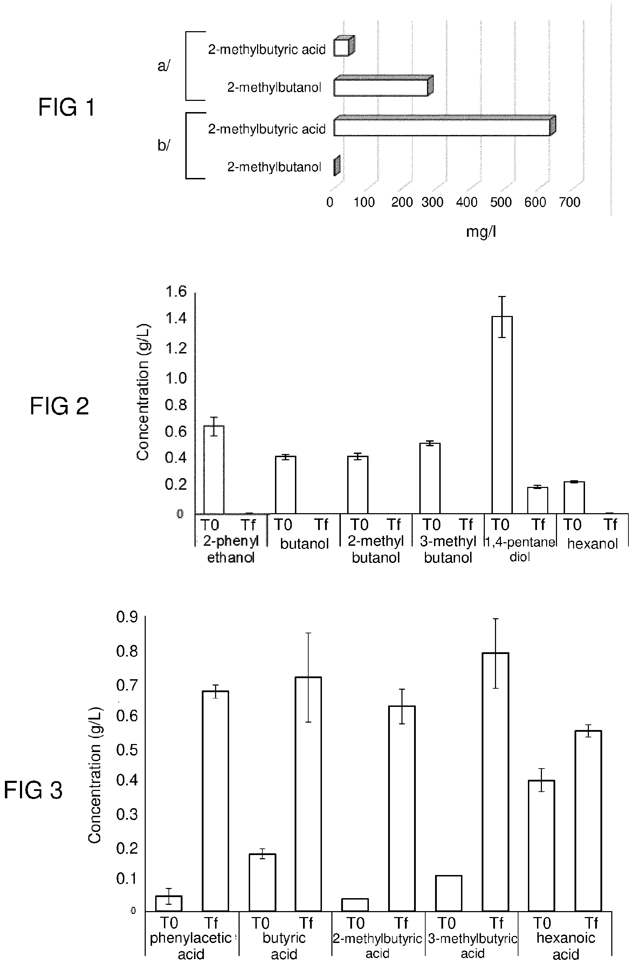

The features and advantages of the invention will emerge more clearly in the light of the following examples of implementation, provided for illustrative purposes only and in no way limitative of the invention, with the support of figures 1 to 5, in which:
- figure 1 represents a bar graph showing the concentrations of produced 2-methylbutyric acid and residual 2-methylbutanol, a/ in the bioconversion medium after 48 h of the selective oxidation step of a method similar to that of the invention with the exception that the bioconversion medium is devoid of glycerol, and b/ in the bioconversion medium after 48 h of the selective oxidation step of a method of producing 2-methylbutyric acid from 2-methylbutanol according to the invention using the bacterial strain *Acetobacter* sp. CIP 58.66;
- figure 2 represents a bar graph showing, for several alcohols, the concentration of the starting alcohol in the bioconversion medium (T0) and at the end (Tf = 48 h) of the selective oxidation step of a method of producing an acid from an alcohol according to the invention, using the bacterial strain *Acetobacter* sp. CIP 58.66;
- figure 3 represents a bar graph showing, for several alcohols, the concentration of the produced acid in the bioconversion medium (T0) and at the end (Tf = 48 h) of the selective oxidation step of a method of producing an acid from an alcohol according to the invention, using the bacterial strain *Acetobacter* sp. CIP 58.66;

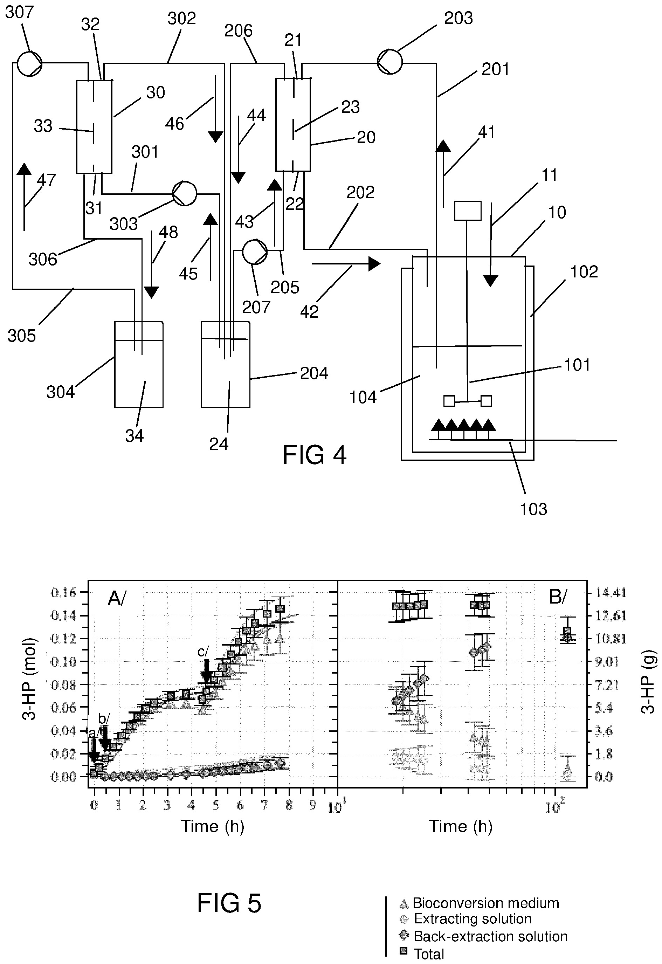

- figure 4 schematically represents a device for implementing a method according to the invention for the production of a carboxylic acid from an alcohol, said method including an acid extraction step and a back-extraction step;
- figure 5 represents graphs showing, as a function of time, during the selective oxidation step of a method of the invention for producing 3-hydroxypropionic acid (3-HP) from 1,3-propanediol by *Acetobacter* sp. CIP 58.66, the concentration of 3-hydroxypropionic acid respectively in the bioconversion medium, in the extracting solution and in the back-extraction (stripping) solution, in A/ in the first 8 h, and in B/ after the selective oxidation step is over, but the extraction and back-extraction continue, the time being represented in logarithmic scale; in this figure, a/ represents a first 1,3-propanediol addition in the bioconversion medium, b/ represents the start of the extraction step and c/ represents a second 1,3-propanediol addition in the bioconversion medium.

### Preparation of the bacterial strain

A lyophilisate of *Acetobacter* sp. CIP 58.66 from the Biological Resource Center of Pasteur Institute (Paris, France) is inoculated into 50 mL of medium comprising 25 g.L⁻¹ mannitol, 5 g.L⁻¹ yeast extract, 3 g.L⁻¹ peptone and a 5.5 mol.L⁻¹ sulfuric acid solution in appropriate quantity to adjust the pH to 6.5, contained in a 250 mL baffled shake-flask. The culture is incubated 1 week at 25°C under stirring at 200 rpm. After 1 week, 2 mL of this culture are used to inoculate 100 mL of fresh medium (20 g.L⁻¹ ethanol, 5 g.L⁻¹ yeast extract, 3 g.L⁻¹ peptone; pH adjusted to 6.5) in a 500 mL baffled shake-flask. This second culture is incubated 1 week in the same conditions. Then glycerol is added to the medium (20 % (w/v)), as cryoprotectant. The broth is aliquoted in 1 mL tubes and stored at -80°C, with a cell density around 9.5.10⁸ cell mL⁻¹. These cryotubes are used as starters for inoculum preparation for all the experiments described below.

For each step of the method described below, the medium is sterilized before use by autoclaving for 20 min at 120°C, then stored at 4°C in a cold room or at room temperature.

The optical density of the samples is measured at 600 nm. Milli-Q water is used for dilution of the samples when required, to stay within the linearity range of the device.

### Example 1 - Bioconversion of 2-methylbutanol into 2-methylbutyric acid

### Growth step

*Acetobacter* sp CIP 58.66 is cultivated in a growth medium containing, per liter, 8.71 g of K₂HPO₄, 5 g of peptone, 3 g of yeast extract and 20 g of glycerol.

After 24 h the bacterium reaches the late exponential phase (optical density at 600 nm close to 3).

### Bioconversion step

Bioconversion with *Acetobacter* sp CIP 58.66 is carried out on a sterile bioconversion medium, in shaken culture, in a 250 ml baffled Erlenmeyer flask containing 25 ml of medium. The medium contains, per liter, 8.71 g of K₂HPO₄, 5 g of peptone, 3 g of yeast extract and either no glycerol or 20 g glycerol. The pH of the medium is adjusted to 6.5 with 5.5 M H₂SO₄. 2-Methyl butanol is sterilized by filtration and added to the sterile medium at the rate of 600 mg/L before inoculation of the bacterium. The medium is seeded at 3% v/v with the bacterial culture obtained at the end of the growth phase.

A 5 ml sample of the bioconversion medium is taken after 48 h. It is acidified with 300 µl of 1M hydrochloric acid to facilitate the extraction of acids. The medium is centrifuged and then the supernatant (3 ml) is acidified with 300 µl of 1N HCl, extracted three times with 1 ml of dichloromethane. The dichloromethane extracts are combined and dried up with anhydrous sodium sulfate.

The extract is analysed by GC-MS (gas chromatography - mass spectrometry) for the presence of 2-methylbutyric acid and 2-methylbutanol, using two different columns.

The alcohol was analysed with a 6890 Agilent GC-MS equipped with an automatic sampler (injecting 1µl CH₂Cl₂ sample extract), The samples were injected in Splitless mode, with ultrapure helium as carrier gas at an initial flow rate of 1.2 mL/min, (Split open after 1 min, 30 ml/min) on a 60 m long BPX 70 capillary column (0.25 mm diameter with a 0.25 µm phase thickness). Oven was programmed from 35 to 60°C (20°C/min) then from 60 to 240°C (3°C/min) and set for 5 min when 240°C was reached. Detection was made with a mass spectrometer quadrupole (Source 230°C, 70MeV). The alcohol was identified from injection of standards on the basis of its retention time and mass spectra. An external standard curve was made from standard solutions made from the initial culture medium extracted and analysed as the samples. Total Ion Current was used for quantification.

The acid was also analysed with the same GC-MS device but with 30 m long FFAP capillary column (0.25 mm diameter with a 0.25 µm phase thickness). Oven was programmed from 40 to 110°C (3°C/min) then from 110 to 250°C (7°C/min). Detection was made with the mass spectrometer as described for the alcohol. Identification and quantification were made as for the alcohol.

The results obtained are shown in Figure 1.

It is observed that in the absence of glycerol in the fermentation medium the bioconversion to 2-methylbutyric acid is low (42 +/- 10 mg/L of acid in the bioconversion medium) and the precursor alcohol is not completely consumed, as there remains 273 +/- 28 mg/L of residual 2-methylbutanol in the bioconversion medium, i.e. almost half of the initial concentration. The molar yield is low (26.5%).

In the presence of glycerol in the bioconversion medium, about 15 times more 2- methylbutyric acid are produced (593 +/- 21 mg/L) and there is no more than 1 mg/L of 2-methylbutanol left in the bioconversion medium. The molar conversion yield is close to 100% (apart from measurement errors, 107% measured).

These results demonstrate that, surprisingly, the molar yield of the bioconversion reaction is much higher when the bioconversion medium is supplied with glycerol, compared to when it is devoid of glycerol.

Furthermore, the mean productivity of the method is also much higher when the bioconversion medium is supplied with glycerol, compared to when it is devoid of glycerol: for a 48 hours reaction in a bioconversion medium containing glycerol, a mean productivity of 13.1 mg.l⁻¹.h⁻¹ is measured; whereas a mean productivity of only 1.58 mg.l⁻¹.h⁻¹ is measured for a 55 hours bioconversion in a bioconversion medium devoid of glycerol.

### Example 2 - bioconversion of other alcohols / diols

### Growth step

The growth medium contains, per liter, 8.71 g of K₂HPO₄, 5 g of peptone, 3 g of yeast extract, 20 g of glycerol and deionised water (qs 1 L). The pH of the medium is adjusted to 6.5 with sulfuric acid 5.5 M.

The growth step is carried out in 500 ml baffled flasks filled with 10% by volume of growth medium and inoculated with 1 cryotube containing the bacterium. The flasks are closed with carded cotton and incubated at 30°C for 48 h, under shaking at 200 rpm. After 48 h the cells have reached late exponential growth phase (optical density at 600nm close to 2.5). 3 ml of the bacterial preculture are collected for the subsequent selective oxidation step of the method.

### Selective oxidation step

The bioconversion medium is the same as the growth medium, with or without 20 g/L of glycerol.

The following alcohols are tested: 2-phenyl ethanol, n-butanol, 2-methyl butanol, 3-methyl butanol, 1,4-pentanediol, hexanol.

Solutions of these alcohols at a concentration of 7.5 g/L, are prepared as follows. 0.375 g of each alcohol is weighed into 50 mL volumetric flasks using a precision balance and glass pipettes. The flasks are then filled up to the mark with Milli-Q water, mixed by manual stirring, and their contents are transferred to sterile 180 mL plastic bottles. The sterility of these solutions is ensured by filtration in 50 ml glass vials, which have been previously autoclaved, using 20 ml syringes and sterile filters having pores with a 0.22 µm diameter.

The selective oxidation step is carried out in 250 ml baffled vials filled to 10% by volume of the bioconversion medium, seeded with 3 ml of bacterial preculture and fed with 2 ml of alcohol solution.

The incubation conditions are the same as in the growth step.

To ensure that there is no contamination, bacterial aliquots are regularly sampled and laid down on solid growth medium contained in sterile Petri dishes of 9 cm diameter. The composition of the solid growth medium is identical to the one of the liquid growth medium but it additionally contains 20 g.L⁻¹ of agar. A 100 µL sample of the culture to be tested at the desired dilution (10⁻⁶, 10⁻⁷, or 10⁻⁸) is placed on the solid growth medium, and distributed using a sterile rake. The dishes are closed and placed in an oven at 30 °C for a few days, checking them regularly to monitor growth and to ensure the absence of contamination.

After 48 hours of culturing, 5 ml samples are collected from the bioconversion medium and acidified with 300 µL of 1 M HCl, to lower the pH to about 2. The cell pellet is separated by centrifugation at 13,400 G at 15°C for 5 min. The supernatant containing the molecules of interest is recovered.

Extraction is carried out by adding 1 mL of CH₂Cl₂ to the recovered supernatant, followed by vortexing for 10 s. The organic phase is recovered with a glass pipette in 4 mL vials. This operation is performed 3 times per sample to maximize the extraction yield. The CH₂Cl₂ phase is then dehydrated with anhydrous sodium sulfate.

Each sample thus obtained is analysed by GC-MS (Gas Chromatography coupled with Mass Spectrometry) using the same materials and methods as described in example 1.

To allow a quantitative assay, 3 external standards comprising the expected compounds at different known concentrations (500, 250, 125 and 62.5 mg/L) are prepared and analysed in parallel with the samples. This makes it possible to correlate the surfaces of the peaks obtained with the respective concentrations of the different molecules.

The constituents separated by chromatography are fragmented and ionized by electronic impact at 70 eV, separated according to their m/z ratio using a quadrupole and finally detected using a high energy conversion dynode. The total electric current proportional to the abundance of ions selected by the quadrupole is then measured by the machine.

These experiments were carried out in triplicate.

The results obtained when glycerol was present in the bioconversion medium are shown on figure 2 (concentration of alcohol in the bioconversion medium at the beginning and after 48 h of the selective oxidation step) and on figure 3 (concentration of produced organic acid in the bioconversion medium at the start and after 48 h of the selective oxidation step).

For 2-phenylethanol, butanol, 3-methylbutanol, 2-methylbutanol and hexanol, a very significant bacterial growth was observed. In each corresponding culture, the added alcohol was exhausted after 48 h of culture. 90 % of 1,4-pentanediol was consumed after 48 h of culture.

Depending on the alcoholic precursor, the concentration of produced acid was between 0.6 and 0.8 g/l, and the productivity was between 12 and 15 mg.l⁻¹.h⁻¹.

### Example 3 - complete integrated process

### Device

The device used for this experiment in shown in figure 4.

It comprises:
- a bioconversion reactor 10 for containing the bioconversion medium 104, equipped with stirring means 101, heating means 102 and aerating means 103;
- a first membrane contactor 20 containing a hollow fibers membrane. The hollow fibers are contained in an external shell, around a central baffle (not shown on the figure);
- means for circulating bioconversion medium 104 from the bioconversion reactor 10 through the shell compartment of the first membrane contactor 20 and back to the bioconversion reactor 10, said means comprising: a first pipe 201 for circulating said liquid from the bioconversion reactor to a first end 21 of the first membrane contactor 20, a second pipe 202 for circulating said bioconversion medium from a second end 22 of the first membrane contactor 20 opposite the first end 21, to the bioconversion reactor, and a first pump 203 for flowing said liquid in the first pipe 201 and in the second pipe 202;
- means for circulating a first liquid solution 24, in countercurrent with respect to the bioconversion medium 104, from a first flask 204 containing said first liquid solution 24, in the hollow fibers of the membrane of the first membrane contactor 20, and back to the first flask 204. Said means comprise: a third pipe 205 for circulating said first liquid solution 24 from the first flask 204 to the second end 22 of the first membrane contactor 20, a fourth pipe 206 for circulating said first liquid solution 24 from the first end 21 of the first membrane contactor 20 to the first flask 204, and a second pump 207 for flowing said first liquid solution 24 in the third pipe 205 and in the fourth pipe 206. In the figure, a dotted line indicated by reference 23 symbolizes the separation between the phases in the first membrane contactor;
- a second membrane contactor 30 containing a hollow fibers membrane. The hollow fibers are contained in an external shell, around a central baffle (not shown on the figure);
- means for circulating the first liquid solution 24 from the first flask 204 through the shell compartment of the second membrane contactor 30 and back to the first flask 204, said means comprising: a fifth pipe 301 for circulating said first liquid solution 24 from the first flask 204 to a first end 31 of the second membrane contactor 30, a sixth pipe 302 for circulating said first liquid solution 24 from a second end 32 of the second membrane contactor 30 opposite the first end 31, to the first flask 204, and a third pump 303 for flowing said first liquid solution 24 in the fifth pipe 301 and in the sixth pipe 302;
- and means for circulating a second liquid solution 34, in countercurrent with respect to the first liquid solution 24, from a second flask 304 containing said second liquid solution 34 , through the hollow fibers of membrane of the second membrane contactor 30, and back to the second flask 304, said means comprising: a seventh pipe 305 for circulating said second liquid solution 34 from the second flask 304 to the second end 32 of the second membrane contactor 30, an eighth pipe 306 for circulating said second liquid solution 34 from the first end 31 of the second membrane contactor 30 to the second flask 304, and a fourth pump 307 for flowing said second liquid solution 34 in the seventh pipe 305 and in the eighth pipe 306. In the figure, a dotted line indicated by reference 33 symbolizes the separation between the phases in the second membrane contactor.

The bioconversion reactor 10 is a 3.6 L Labfors 4 bioreactor (Infors), with its associated software (Iris v.5), for data acquisition and process control. The stirring means 101 equipping it are a single Rushton turbine for broth stirring. The aerating means 103, for providing air into the bioconversion medium contained in the reactor 10, comprise a mass air flowmeter. The heating means 102 are formed of a double wall surrounding the reactor 10, in which hot water circulates.

The first hollow fibres membrane contactor 20 and the second hollow fibres membrane contactor 30 are identical. They include a 2.5 x 8 Liqui-Cel® module containing X50 fibres (Membrana, USA). Characteristics of the module are as follows: material: polypropylene, internal diameter 58.4 mm, internal length 203 mm, number of fibres 9800, contact area 0.4 m². The characteristics of the fibers are as follows: material: polypropylene, internal diameter 220 µm, external diameter 300 µm, effective length 146 mm, wall thickness 40 µm, porosity 40%, average pore size 0.03 µm.

### Primary growth step on glycerol

Biocatalyst production is performed in batch mode, with glycerol as growth substrate, in the reactor 10. An initial volume of 1.2 L of a growth medium B2 containing 5 g/L of yeast extract, 3 g/L of peptone, 10 g/L of glycerol, of pH 5.0, is inoculated with the bacterial pre-culture, so that the initial biomass concentration is 0.02 cell dry weight per liter. Due to the inoculum addition, initial pH rises from 5.0 to 5.2. During bacterial growth, pH is left uncontrolled, but agitation speed (from 100 to 800 rpm) and airflow rate (from 1 to 4 NL min-1) are automatically controlled in order to maintain the partial oxygen pressure in the reactor (pO₂) above 40 %. The temperature of the growth medium is 30 °C.

In all the experiment, the Cell dry weight (CDW) is determined by optical density (OD) measurement, and according to the formula: CDW = 0.59 x OD.

When the late exponential phase is reached the cell dry weight is around 0.88 (+/- 0.05) g/L, corresponding to an optical density at 600 nm of around 1.49.

### Fed-batch selective oxidation step

When late exponential phase is reached, after 32 h of growth step, 6 mL of filter-sterilized 1,3-propanediol (98 % w/v) is added to the medium in order to trigger bioconversion, as indicated by reference 11 on figure 4. The bioconversion medium 104 thus obtained contains 5 g/l of 1,3-propanediol and 8,6 g/l of glycerol, that has not been consumed during the growth step.

The selective oxidation step is carried out at 30 °C with partial oxygen pressure (pO₂) controlled at 40 %. The bacterial cells produce 3-hydroxypropionic acid by oxidizing 1,3-propanediol.

### Extraction and back-extraction steps

At the end of the growth step on glycerol the pH of the medium is close to 7. An acidification of the bioconversion medium occurs after the 1,3-propanediol addition, due to production by the bacterium of 3-hydroxypropionic acid. The bioconversion medium 104 is brought in contact with the extracting solution only after pH has reached 4.6, which is close to the pKa of 3-hydroxypropionic acid (4.51). The extraction is started 0,45 h after the 1,3-propanediol addition. No base is added for pH control, and pH varies freely according to production and extraction rates.

The extracting solution (first liquid solution 24) consists of 20 % (v/v) of didodecylmethylamine (DDMA) diluted in 40 % (v/v) dodecanol and 40 % (v/v) dodecane. The corresponding partition coefficient, i.e. the ratio, at equilibrium, between the 3-hydroxypropionic acid concentration in the organic phase (extracting solution 24) and in the aqueous phase (bioconversion medium 104) and viscosity are determined as described in the publication of Sanchez-Castañeda et al. 2020, Journal of Chemical Technology and Biotechnology 95:1046-1056 and are equal to 0.78 and 4.7 mPa.s, respectively.

For back-extraction, a second liquid solution 34, also called stripping solution, containing NaOH 0.5 mol.L⁻¹ in water, is used.

The method is implemented as follows.

Before each experiment, the membrane contactors 20, 30 have been washed by circulating a 60 % (v/v) isopropanol solution for 2 hours through the fibres and the shell side, then drained and rinsed with sterilized Reverse Osmosis (RO) water and finally dried overnight by flushing compressed air.

The bioconversion medium 104 is continuously circulated at a flow rate of 450 ml/min in the first pipe 201 in the direction indicated by arrow 41 on figure 4, through the shell of the first membrane contactor 20, then in the second pipe 202, in the direction indicated by arrow 42, back to the bioconversion reactor 10. At the same time, the extracting solution 24 is continuously circulated at a flow rate of 430 ml/min from the first flask 204, in the third pipe 205 in the direction indicated by arrow 43 on figure 3, in the hollow fibers of the first membrane contactor 20, in countercurrent flow with respect to the bioconversion medium 104, then in the fourth pipe 206, in the direction indicated by arrow 44, back to the first flask 204.

The central baffle of the first membrane contactor 20 forces a radial flow in the shell compartment, between the fibres that are woven together. The membrane being hydrophobic, the pores are filled with the organic phase (extracting solution 24), which is therefore in direct contact with the aqueous phase (bioconversion medium 104).

3-hydroxypropionic acid is thereby extracted from the bioconversion medium 104 into the extracting solution 24, through an organic acid-base interaction. The extracting solution 24 gets gradually loaded with this acid. Glycerol and 1,3-propanediol are not affected by the extraction step, neither are the bacterial cells contained in the bioconversion medium 104.

For recovery of the 3-hydroxypropionic acid from the extracting solution 24, a back-extraction is performed in the second membrane contactor 30.

To this end, 1 L of the second liquid solution 34 is continuously circulated inside the fibers of the second membrane contactor 30, while the extracting solution 24 is continuously circulated in the shell of the second membrane contactor 30.

More precisely, the extracting solution 24 is continuously circulated at a flow rate of 430 ml/min in the fifth pipe 301 in the direction indicated by arrow 45 on figure 4, through the shell of the second membrane contactor 30, then in the sixth pipe 302, in the direction indicated by arrow 46, back to the first flask 204. At the same time, the stripping solution 34 is continuously circulated at a flow rate of 450 ml/min from the second flask 304, in the seventh pipe 305 in the direction indicated by arrow 47 on figure 4, in the hollow fibers of the second membrane contactor 30, in countercurrent flow with respect to the extracting solution 24, then in the eighth pipe 306, in the direction indicated by arrow 48, back to the second flask 304.

3-hydroxypropionic acid is thereby gradually transferred from the extracting solution 24 to the aqueous stripping solution 34, in the form of a sodium salt.

After 4.6 h of bioconversion, a new 6 mL addition of 1,3-propanediol in the bioconversion medium is performed.

The selective oxidation step is carried out for 8 h. Extraction and back-extraction are continued for a further 100 h.

Samples are taken from the bioconversion medium, the extracting solution and the stripping (back-extraction) solution at different times, for components quantification. Biomass evolution is also monitored in the bioconversion reactor, by off-line optical density measurements.

### Results

Concentrations of 3-hydroxypropionic acid, 1,3-propanediol and glycerol are quantified through HPLC (high-performance liquid chromatography). The samples are prepared differently for aqueous (bioconversion medium and stripping phase) and organic (extracting phase) samples, as follows:
- bioconversion medium samples: proteins are precipitated with trichloroacetic acid, then the samples are centrifuged and only supernatants are recovered. The samples are then filtrated with a nylon filter (0.22 µm pore diameter);
- extracting phase samples: these samples are first back-extracted overnight with an equal volume of NaOH (0.5 mol/L), then the aqueous phase is recovered and filtered (nylon filter, 0.22 µm pore diameter);
- stripping phase samples: these samples are filtered (nylon filter, 0.22 µm pore diameter).

All samples are analyzed on an Aminex® HPX-87H exchange column and a refractive index detector, with H₂SO₄ as mobile phase. For 3-hydroxypropanal, the analysis conditions are 35°C, H₂SO₄ at 0.6 mL/min and 5 mmol/L. For 3-hydroxypropionic acid, 1,3-propanediol and glycerol the conditions are: 65°C, H₂SO₄ at 0.4 mL/min and 0.5 mmol/L.

At the end of the growth step, after 32 h of growth, Cell dry weight (CDW) concentration reaches 0.98 +/- 0.12 g_{CDW} L⁻¹, with a corresponding yield of 0.42 +/- 0.12 g_{CDW}.g⁻¹_{gycerol}. Only 21.1+/- 3.3 % of the initial glycerol was consumed. No by-product of glycerol consumption, such as organic acids, can be detected in the medium.

During the selective oxidation step, both 1,3-propanediol inputs, at 0 h and 4.6 h of the bioconversion step, can be associated with a new exponential growth phase, in all replicates. Numbers of generations (Ng) are similar after both inputs, and overall, CDW increases from 1.01 +/- 0.06 to 2.75 +/- 0.07 g_{CDW}.L⁻¹. The pH decreases from 6.9 to 4.2, and it decreases even further, until 3.9, after the second 1,3-propanediol addition.

During the selective oxidation step, glycerol is further consumed, but never fully depleted: after 25 h, 37.9 +/- 2.9 % of the glycerol initially supplied was consumed, instead of 21.1 +/- 3.3 % at the beginning of the selective oxidation step.

While glycerol is likely the main growth substrate, the presence of 1,3-propanediol in concentrations under 10 g.L⁻¹ is also shown to enhance the growth of *Acetobacter* sp. CIP 58.66 on this complex medium.

The results obtained in the bioconversion reactor are shown in table 1.

**Table 1 - Performance of a method of the invention for producing 3-hydroxypropionic acid from 1,3-propanediol**

| | 1^{st} alcohol addition | | 2^{nd} alcohol addition | |
|---|---|---|---|---|
| Time (h) | 0 | 4.46 | 4.61 | 7.63 |
| Growth phase | 1^{st} exponential | | 2^{nd} exponential | |
| CDW (g/L) | 1.01 | 1.57 | 1.56 | 1.99 |
| Maximal specific growth rate | 0.17 | | 0.08 | |
| Total acid production (g) | 0 | 6.05 | 6.65 | 13.00 |
| Acid yield (mol_{acid}/mol_{alcohol}) | - | 0.83 | 0.90 | 0.91 |
| Maximal specific acid productivity (g_{acid}/(g_{CDW}/h)) | 2.36 | | 2.58 | |
| Average specific acid productivity (g_{acid}/(g_{CDW}/h)) | 1.11 | | 1.12 | |

The physiological state of *Acetobacter* sp. CIP 58.66 is monitored during the selective oxidation step, by dual fluorescent staining and flow cytometry analysis. For each sample, cells can be divided into three sub-populations: (i) enzymatically active cells, (ii) altered cells, that are still enzymatically active, but whose membranes are deteriorated, and (iii) dead cells. During the first 4 h of the selective oxidation step, the proportion of enzymatically active cells remains between 75 and 87 %, while dead cells represent between 11 and 23 % of the overall population. After 4 h of the selective oxidation step, the proportion of enzymatically active cells decreases during approximately 2 h, before reaching values between 46 and 55 % at the end of the selective oxidation step. Overall, the biocompatibility of the system is very satisfactory, since half of the bacterial population is still active after 7.63 h of the selective oxidation step, when 1,3-propanediol is almost fully depleted. Interestingly, between 18 and 25 h after the selective oxidation step starts, the proportion of enzymatically active cells remains between 14 and 17 %. This demonstrates that this process setup is suitable for continuous extractive bioconversion, for at least 25 h.

For both 1,3-propanediol additions, 3-hydroxypropionic acid is the main product, with an overall yield of 0.91+/- 0.06 mol_{acid}.mol⁻¹_{alcohol}. The maximal specific productivities (q_{acid,max}) that are reached for each successive phases are similar.

A slight and transient accumulation of the corresponding intermediate product 3-hydroxypropanal is detected around 1 h after each alcohol input, without exceeding 0.20 +/- 0.11 and 0.24 +/- 0.14 g.L⁻¹, respectively.

3-hydroxypropionic acid distribution among the bioconversion medium, the extracting solution and the stripping solution, is shown in Figure 5.

As can be seen on this figure, during the selective oxidation step extraction starts slowly, leading to a progressive 3-hydroxypropionic acid accumulation in the extracting solution. The extraction rate increases progressively with 3-hydroxypropionic acid production, and back-extraction in the stripping solution starts later: 3-hydroxypropionic acid is detected in the stripping solution only after 1.7 h. Back-extraction rate increases as acid accumulates in the extracting solution, eventually exceeding the extraction rate, as shown by the decrease in 3-hydroxypropionic acid in the extraction solution (in B in the figure). After 8 h of bioconversion, all the added 1,3-propanediol is consumed and 3-hydroxypropionic acid production stops. From then on, 3-hydroxypropionic acid concentration decreases both in the bioconversion reactor and in the extracting solution, while the concentration in the stripping solution increases. The system is left running in order to recover all the produced 3-hydroxypropionic acid. After 114 h, nearly all the 3-hydroxypropionic acid is recovered in the stripping solution, thus achieving therein a final concentration in 3-hydroxypropionic acid of 12.63+/-0.50 g.L⁻¹, while almost no 3-hydroxypropionic acid is detected in the bioconversion medium and in the extracting solution.

Higher 3-hydroxy propionic acid concentrations can be reached easily by reducing the back-extraction phase volume:
It is observed that the overall effect of the pertraction system of the invention is well tolerated by *Acetobacter* sp. CIP 58.66 during bioconversion, and that 3-hydroxypropionic acid can be produced with high yield by the method of the invention, leading to recovery of this acid with a high purity degree in the stripping solution.

The mean productivity, for the first 8 hours of the selective oxidation step, is 1.53 g/(L.h), with respect to the working volume of the reactor.

## Claims

1. Method for the production of a carboxylic acid from a primary alcohol, said method being **characterized in that** it comprises:
- a first step of growing a strain of bacterium of the genus *Acetobacter* capable of selectively oxidizing said alcohol into said carboxylic acid, in an appropriate growth medium comprising glycerol as carbon source, until said bacterium reaches a late exponential growth phase;
- then a selective oxidation step comprising aerobically culturing said bacterium in a bioconversion reactor containing an appropriate liquid bioconversion medium containing said alcohol, for a sufficient time to oxidize said alcohol into said carboxylic acid, said bioconversion medium containing glycerol.

2. Method according to claim 1, wherein said bioconversion medium contains a concentration of between 5.5 and 20 g/l of glycerol.

3. Method according to any of claims 1 or 2, wherein said growth medium contains an initial concentration of between 10 and 20 g/l of glycerol.

4. Method according to any of claims 1 to 3, wherein said bioconversion medium contains between 0.5 and 10 g/L, preferably between 2 and 5 g/L, of said alcohol.

5. Method according to any of claims 1 to 4, wherein said selective oxidation step comprises batch-feeding said alcohol into said bioconversion reactor.

6. Method according to any of claims 1 to 5, wherein said alcohol is chosen in the group consisting of 1,3-propanediol, 2-methylbutanol, 2-phenylethanol, butanol, 1,4-butanediol, 3-methylbutanol, 1,4-pentanediol and hexanol.

7. Method according to any of claims 1 to 6, wherein said selective oxidation step is carried out for at least 4 hours.

8. Method according to any of claims 1 to 7, comprising adjusting the pH of said bioconversion medium at a value comprised between 4 and 5.

9. Method according to any of claims 1 to 8, comprising the extraction of said carboxylic acid from said bioconversion medium.

10. Method according to claim 9, wherein said extraction of the carboxylic acid from said bioconversion medium is carried out continuously.

11. Method according to any of claims 9 or 10, wherein said extraction of the carboxylic acid from said bioconversion medium is a liquid-liquid reactive extraction using an amine-containing organic extracting solution.

12. Method according to claim 11, wherein said liquid-liquid reactive extraction is carried out outside the bioconversion reactor.

13. Method according to claim 12, wherein said liquid-liquid reactive extraction is carried out through a membrane, preferably in a hollow-fibre membrane contactor.

14. Method according to any of claims 11 to 13, comprising a step of recovering said carboxylic acid from said extracting solution.

15. Method according to any of claims 1 to 14, comprising a preliminary step of bioproducing said alcohol by a micro-organism and a step of feeding the alcohol thus produced into said bioconversion medium without any intermediate purification step.
